# EUROPEAN PATENT SPECIFICATION

(11) **EP 1 437 131 B1**
(45) Date of publication and mention of the grant of the patent: **25.04.2012**
(21) Application number: 04006330.7
(22) Date of filing: 25.08.2000
(51) Int. Cl.: A61K 31/403, A61P 3/10

(54) **Use of ramipril for the prevention of diabetes in a patient with no preexisting congestive heart failure**
Verwendung von Ramipril zur Vorbeugung von Diabetes bei einem Patienten ohne bestehendes kongestives Herzversagen
Utilisation de ramipril pour la prévention du diabète chez un patient sans insuffisance cardiaque congestive préexisitante

(30) Priority: 27.08.1999 SE 9903028
(43) Date of publication of application: 14.07.2004
(62) Divisional of application: 00965898.0
(73) Proprietor: Sanofi-Aventis Deutschland GmbH, 65929 Frankfurt am Main (DE)
(72) Inventor: Schoelkens, Bernward, Prof. Dr., 65779 Kelkheim (DE); Bender, Norbert, Dr., 65719 Hofheim (DE); Rangoonwala, Badrudin, Dr., 65719 Hofheim (DE); Dagenais, Gilles, St.-Nicholas, Quebec, J7A 3P8 (CA); Gerstein, Hertzel, Hamilton, Ontario L8S 4A8 (CA); Ljunggren, Anders, 43163 Mölndal (SE); Yusuf, Salim, Carlisle, Ontario L0R 1H2 (CA)

(56) References cited:
- EP-A- 0 331 014
- EP-A- 0 426 066
- EP-A- 0 482 498
- WO-A-01/15674
- DE-A- 4 308 504
- US-A- 5 190 970
- HANSSON L ET AL: "Effect of angiotensin-converting-enzyme inhibition compared with conventional therapy on cardiovascular morbidity and mortality in hypertension: the Captopril Prevention Project (CAPPP) randomised trial" LANCET, XX, XX, vol. 353, no. 9153, 20 February 1999 (1999-02-20), pages 611-616, XP004266024 ISSN: 0140-6736
- YUSUF S ET AL: "EFFECTS OF AN ANGIOTENSIN-CONVERTING-ENZYME INHIBITOR, RAMIPRIL, ON CARDIOVASCULAR EVENTS IN HIGH-RISK PATIENTS" NEW ENGLAND JOURNAL OF MEDICINE, THE, MASSACHUSETTS MEDICAL SOCIETY, WALTHAM, MA, US, vol. 342, no. 3, 20 January 2000 (2000-01-20), pages 145-153, XP008013952 ISSN: 0028-4793
- HSUEH W A: "In diabetes, treat hidden heart disease." CLEVELAND CLINIC JOURNAL OF MEDICINE. UNITED STATES NOV 2000, vol. 67, no. 11, November 2000 (2000-11), pages 807-808, 809 - 810, 813, XP008030409 ISSN: 0891-1150

## Description

### FIELD OF INVENTION

The present invention relates to use of the use of ramipril or a pharmaceutically acceptable salt thereof in the manufacture of a medicament for the prevention or reduction of onset of diabetes in a patient with no preexisting congestive heart failure (CHF) and where the patient is at high risk for a cardiovascular event due to a history of previous ischemic heart disease, stroke or peripheral arterial disease.

### BACKGROUND OF THE INVENTION

Compounds that interfere with the RAS are well known in the art and are used to treat cardiovascular diseases, particularly arterial hypertension and heart failure. Principally, the RAS can be interferred with by inhibition of the enzymes synthesizing angiotensins or by blocking the corresponding receptors at the effector sites. Available today are inhibitors of the angiotensin converting enzyme (ACE) and angiotensin II type 1 receptor (AT II) antagonists.

ACE inhibitors are compounds which inhibit the conversion of angiotensin I into the active angiotensin II as well as the breakdown of the active vasodilator bradykinin. Both of these mechanisms lead to vasodilation. Such compounds have been described in, for example, EP 158927, EP 317878, US 4,743,450, and US 4,857,520.

Ramipril (disclosed in EP-A-079022) is a long-acting ACE inhibitor. Its active metabolite is the free diacid ramiprilat, which is obtained in vivo upon administration of ramipril. In hypertensive patients administration of ramipril is known to cause a reduction in peripheral arterial resistance and thus a reduction of the blood pressure without a compensatory rise in heart rate. It is currently being used in the treatment of hypertension and CHF. Furthermore, ramipril has been shown to reduce mortality in patients with clinical signs of congestive heart failure after surviving an acute myocardial infarction. Ramipril has been suggested to have an added advantage over many other ACE inhibitors due to its pronounced inhibition of ACE in tissues resulting in organ protective effects in e.g. the heart, kidney, and blood vessels.

Compounds that interfere with the RAS including ACE inhibitors and AT II antagonists are currently used in the treatment of various cardiovascular disorders, especially in patients exhibiting a high blood pressure. Use of said compounds in prevention of cardiovascular disorders is much less common and the use of said compounds in the prevention of stroke, diabetes and /or CHF is hitherto unknown.

### SUMMARY OF THE INVENTION

The present invention relates to claim 1 especially in patients exhibiting normal or low blood pressure.

### DETAILED DESCRIPTION OF THE INVENTION

It has surprisingly been found that diabetes can be prevented by use of ramipril. The present invention is especially surprising in that especially patients with an essentially maintained heart function and/or exhibiting a normal or low blood pressure benefit markedly from the preventive action of ramipril.

Patients exhibiting a normal or low blood pressure are known as normotensive patients. Examples of guidelines defining blood pressure values for different patient groups including different ages, include guidelines issued by the WHO and JNC (USA). In the present invention, a suitable definition of a normal or low blood pressure can be found in JNC VI.

In the present invention, "diabetes" refers to type II diabetes, also known as non-insulin-dependent diabetes mellitus (NIDDM).

### Pharmaceutical formulations

Described are pharmaceutical formulations comprising ramipril or a pharmaceutically acceptable salt as active ingredient, including metabolites, for use in the prevention or reduction of onset of diabetes.

For clinical use, ramipril is formulated into a pharmaceutical formulation for oral, intravenous, subcutaneous, tracheal, bronchial, intranasal, pulmonary, transdermal, buccal, rectal, parenteral or some other mode of administration. The pharmaceutical formulation may contain the inhibitor in admixture with a pharmaceutically acceptable adjuvant, diluent and/or carrier.

In the preparation of the pharmaceutical formulations the active ingredient may be mixed with solid, powdered ingredients, such as lactose, saccharose, sorbitol, mannitol, starch, amylopectin, cellulose derivatives, gelatin, or another suitable ingredient, as well as with disintegrating agents and lubricating agents such as magnesium stearate, calcium stearate, sodium stearyl fumarate and polyethylene glycol waxes. The mixture may then be processed into granules or pressed into tablets.

The active ingredient may be separately premixed with the other, non-active ingredients, before being mixed to form a formulation.

Soft gelatine capsules may be prepared with capsules containing a mixture of the active ingredient of the invention, vegetable oil, fat, or other suitable vehicle for soft gelatine capsules. Hard gelatine capsules may contain granules of the active ingredients. Hard gelatine capsules may also contain the active ingredients in combination with solid powdered ingredients such as lactose, saccharose, sorbitol, mannitol, potato starch, corn starch, amylopectin, cellulose derivatives or gelatine.

Dosage units for rectal administration may be prepared (i) in the form of suppositories which contain the active substance mixed with a neutral fat base; (ii) in the form of a gelatine rectal capsule which contains the active substance in a mixture with a vegetable oil, paraffin oil or other suitable vehicle for gelatine rectal capsules; (iii) in the form of a ready-made micro enema; or (iv) in the form of a dry micro enema formulation to be reconstituted in a suitable solvent just prior to administration.

Liquid preparations may be prepared in the form of syrups or suspensions, e.g. solutions or suspensions containing the active ingredients and the remainder consisting, for example, of sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents. Liquid preparations may also be prepared in the form of a dry powder to be recon-stituted with a suitable solvent prior to use.

Solutions for parenteral administration may be prepared as a solution of a formulation of the invention in a pharmaceutically acceptable solvent. These solutions may also contain stabilizing ingredients, preservatives and/or buffering ingredients. Solutions for parenteral administration may also be prepared as a dry preparation to by reconstituted with a suitable solvent before use.

The total amount of active ingredient suitably lies in the range of from about 0.1 % (w/w) to about 95 % (w/w) of the formulation, suitably from 0.5 % to 50 % (w/w) and preferably from 1 % to 25 % (w/w).

The pharmaceutical formulations may contain between about 0.1 mg and about 1000 mg of active ingredient, preferably between 1 mg and 100 mg of active ingredient.

The dose of the active ingredient to be administered will depend on the relevant indication, the age, weight and sex of the patient and may be determined by a physician. The dosage will suitably be in the range of from about 0.01 mg/kg to about 20 mg/kg, preferably between 0.1 mg/kg and 10 mg/kg.

The typical daily dose of the active ingredient varies within a wide range and will depend on various factors such as the relevant indication, the route of administration, the age, weight and sex of the patient and may be determined by a physician. In general, dosages, and especially oral and parenteral dosages, will be in the range of from about 0.1 to about 100 mg per day of active ingredient, preferably between 1 and 50 mg per day of active ingredient.

The following Example is intended to illustrate the invention.

### EXAMPLE

A large-scale clinical trial was designed to examine the effect of the ACE inhibitor ramipril versus placebo in reducing cardiovascular events.

The study was conducted in 267 centres in 19 countries over a six year period and included 9,541 participants who are at high risk for cardiovascular events due to a history of previous ischaemic heart disease, stroke, peripheral arterial disease or individuals with diabetes.

The systolic blood pressure at inclusion of the patients was on average 138 mm Hg and thus the patients were normotensive at study start. After one month of therapy with either ramipril or placebo, the systolic blood pressure had decreased by 5.48 mm Hg and 1.59 mm Hg, respectively.

The primary endpoint of the study was myocardial infarction (MI), stroke and cardiovascular (CV) death (mortality).

The study was stopped early because of a very clear reduction in the combined endpoint of cardiovascular deaths, heart attacks and strokes in patients taking ramipril. In addition to the above benefits, there was also a reduction of between a fourth and a fifth in the need for revascularisation procedures (such as coronary artery bypass graft surgery, balloon angioplasty, etc.) and diabetic complications.

There was a clear 32% reduction in the ramipril group in the number of patients who developed a stroke, and this is surprising since patients were normotensive when recruited to the study.

The number of patients who developed CHF was significantly reduced by 21% in the ramipril group, which is unexpected since patients had no signs or symptoms of CHF at study start.

Equally surprising is the marked 36% reduction in the number of patients who developed diabetes in the ramipril group.

### Abbrevations

ACE = angiotensin converting enzyme
AT II = angiotensin II type 1 receptor
CHF = congestive heart failure
IDMM = insulin-dependent, diabetes mellitus
JNC = Joint National Committee
MI = myocardial infarction
NIDDM = non-insulin-dependent diabetes mellitus
WHO = World Health Organization

## Claims

1. The use of ramipril or a pharmaceutically acceptable salt thereof in the manufacture of a medicament for the prevention or reduction of onset of diabetes in a patient with no preexisting congestive heart failure (CHF) and where the patient is at high risk for a cardiovascular event due to a history of previous ischemic heart disease, stroke or peripheral arterial disease

2. The use according to claim 1, where the patient exhibits normal or low blood pressure.

## Patentansprüche

1. Verwendung von Ramipril oder eines pharmazeutisch verträglichen Salzes davon zur Herstellung eines Arzneimittels für die Verhinderung oder Verminderung des Ausbruchs von Diabetes bei einem Patienten ohne vorher vorliegende Stauungsinsuffizienz (CHF) und wobei der Patient ein hohes Risiko für ein cardiovaskuläres Ereignis aufgrund einer Historie von früherer ischämischer Herzerkrankung, Schlaganfall oder peripherer arterieller Erkrankung aufweist.

2. Verwendung nach Anspruch 1, wobei der Patient einen normalen oder niedrigen Blutdruck zeigt.

## Revendications

1. Utilisation de ramipril ou d'un sel pharmaceutiquement acceptable de celui-ci dans la fabrication d'un médicament destiné à la prévention ou à la réduction de l'apparition du diabète chez un patient sans insuffisance cardiaque congestive (CHF) préexistante et lorsque le patient présente un risque élevé d'accident cardio-vasculaire en raison d'antécédents de maladie cardiaque ischémique, d'accident vasculaire cérébral ou de maladie artérielle périphérique.

2. Utilisation selon la revendication 1, lorsque le patient présente une tension artérielle normale ou basse.
